# EUROPEAN PATENT APPLICATION

(11) **EP 4 628 491 A1**
(43) Date of publication of application: **08.10.2025**
(21) Application number: 24382352.3
(22) Date of filing: 05.04.2024
(51) Int. Cl.: C07D 487/04

(54) **SYNTHESIS OF INTERMEDIATES FOR THE PREPARATION OF NEUROKININ-3 RECEPTOR ANTAGONISTS**

(71) Applicant: Química Sintética, S.A., 28805 Alcalá de Henares (ES)
(72) Inventor: Barreca, Giuseppe, 28100 Novara (IT); Ronzoni, Silvano, 28100 Novara (IT); Falzoni, Marianna, 28100 Novara (IT); Ferrante, Luca, 28100 Novara (IT); Fornara, Matthieu, 28100 Novara (IT); Taddei, Maurizio, 53100 Siena (IT); Vinciarelli, Giorgia, 53100 Siena (IT)
(74) Representative: Ponti & Partners, S.L.P

(57) **Abstract**

The present invention relates to synthetic processes for the preparation of compounds having activity as neurokinin-3-receptor agonists, among which Fezolinetant, and of intermediates useful in the synthesis thereof.

## Description

### Field of the invention

The present invention relates to synthetic processes for the preparation of compounds having activity as neurokinin-3-receptor agonists, among which Fezolinetant, and of intermediates useful in the synthesis thereof.

### Background art

Patents EP 2,552,920 B1 and EP 2,948,455 B1 disclose neurokinin-3-receptor agonists of Formula I (among others), where Ar¹ is an optionally substituted 5- or 6-membered aryl or heteroaryl group, R¹ is methyl, ethyl, n-propyl, hydroxyethyl, methoxyethyl, trifluoromethyl, difluoromethyl or fluoromethyl, R² is methyl, ethyl, methoxymethyl, trifluoromethyl, difluoromethyl, fluoromethyl, 1-fluoroethyl, 1,1-difluoroethyl or 2,2,2-trifluoroethyl and " *--- " stands for the (R)-enantiomer or for the racemate of compound of Formula I. In particular, these compounds include compound la Fezolinetant (CAS number 1629229-37-3, *(4-fluorophenyl)-[(8R)-8-methyl-3-(3-methyl-1,2,4-thiadiazol-5-yl)-6,8-dihydro-5H-[1,2,4]triazolo[4,3-a]pyrazin-7-yl]methanone* ), which is a drug substance approved and marketed in USA for the treatment of hot flushes in postmenopausal women and in clinical trials for other sex hormone-related diseases.

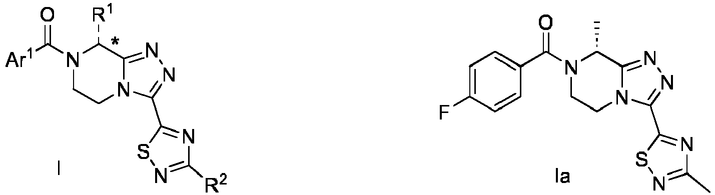

Synthetic methods for the synthesis of such compounds and of Fezolinetant in particular essentially follow two different approaches, disclosed in EP 2,552,920 B1, EP 2,948,455 B1, EP 2,763,992 B9, EP 3,197,876 B1 and illustrated in the scheme below (where the symbols have the same meaning as above detailed).

When following route (a), starting intermediate II is converted to protected intermediate III where PG is a nitrogen protecting group, which is exemplified in the mentioned documents as *tert*-butoxycarbonyl (Boc), allyl, DPP, SES, dimethoxybenzyl (DMB), p-methoxybenzyl (PMB) and generic sp³ protecting groups; for a person skilled in the art it is obvious to consider the use of many other protecting groups among which benzyloxycarbonyl (Cbz) or several other described *e.g*. in Theodora W. Green, Protective Groups in Organic Synthesis, John Wiley & Sons (1999). The resulting intermediate is reacted with triethyloxonium tetrafluoborate to produce IV, which is then reacted with hydrazide derivative IX to obtain V. The protecting group on this last intermediate is finally removed to obtain amine VI which is converted to desired I by amidation.

Route (b) entails instead direct amidation of starting intermediate II, followed by subsequent reactions with triethyloxonium tetrafluoborate and hydrazide IX to obtain I without the need of protecting/deprotecting steps.

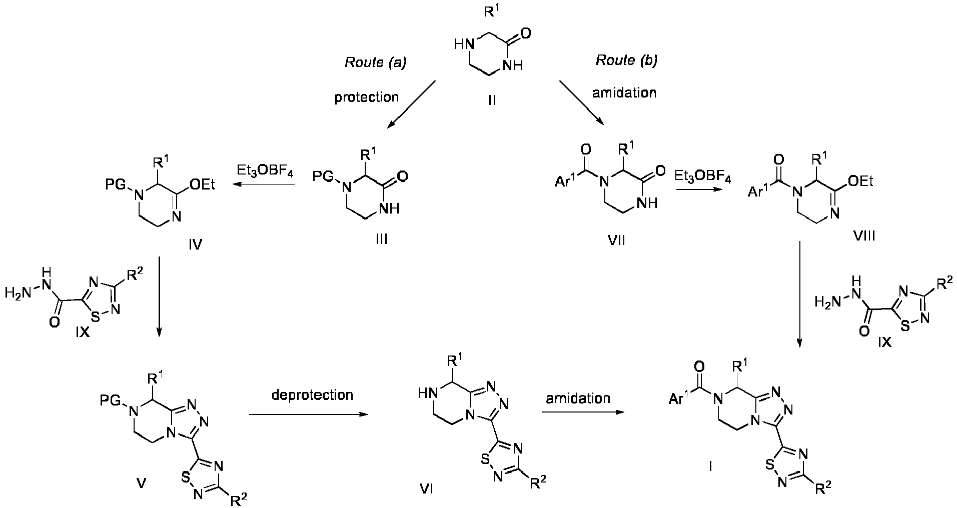

Both routes depend on the use of thiazolecarbohydrazide IX; in patent application EP 3,898,602 A1 a procedure for its preparation when R² is CH₃ or CD₃ is reported, which is stated to improve on the previously known synthesis. Such procedure nonetheless entails the use of toxic gas carbon monoxide in a pressurized reactor, or of unstable and expensive organolithium reagents which have to be used at very low temperature (-65°C) and in strictly inert atmosphere. Besides, very toxic and hazardous hydrazine is also used in the final step of manufacturing of IX.

These conditions are very undesirable when scaling to industrial manufacturing, and the need arises to provide a safer and scalable synthetic method for compounds I which avoids the use of intermediates IX.

### Brief description of the invention

This need is solved by the present invention, which in a first aspect relates to a process for the preparation of compounds of formula X: 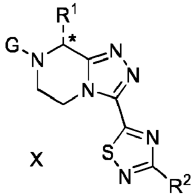 where
- G is: - a -X-Ar¹ group, where X is a carbonyl group CO and Ar¹ is an optionally substituted 5- or 6-membered aryl or heteroaryl group, or - a nitrogen protecting group PG; the nitrogen protecting group PG can be selected from the group including, but not limited to, optionally substituted carbamate groups, optionally substituted alkyl groups, optionally substituted benzyl groups, optionally substituted amide groups; or - a hydrogen;
- R¹ is: an optionally substituted C₁-C₆ alkyl;
- R² is: an optionally substituted C₁-C₆ alkyl; or optionally substituted phenyl group; and

- *---: represents one enantiomer R or S of compounds of formula X or a mixture of the two enantiomers in any proportion including the racemate;
the process including the following step: 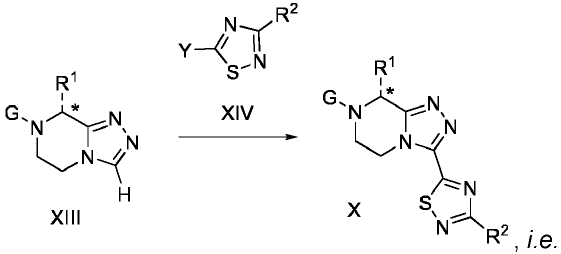 reacting a compound of formula XIII with a compound of formula XIV in a suitable solvent in the presence of one or more metal-based catalysts, optionally in the presence of one or more ligands and/or one or more bases, to obtain a compound of formula X, where Y is a suitable leaving group. The leaving group can be an halogen (Cl, Br or I), a sulfonate group (*e.g.* CH₃SO₂-O-, pTolSO₂-O-, CF₃SO₂-O- or other alkyl- or aryl-sulfonates), a sulfone group (*e.g.* CH₃SO₂-, pToISO₂-, CF₃SO₂- or other alkyl- or aryl-sulfones), a sulfoxide group (*e.g.* CH₃SO-, pToISO-, CF₃SO- or other alkyl- or aryl-sulfoxides), a sulfinate group (*e.g.* CH₃SO-O-, pToISO-O-, CF₃SO-O- or other alkyl- or aryl-sulfinates), and the other symbols G, R¹, R² and " *--- " have the same meaning as described above.

Optionally, the process may also include one or more of the following steps to prepare compounds of formula XIII or transform compounds of formula X into other compounds with the same general formula but different specific meanings of the symbols:
i) reacting a compound of formula XII with formohydrazide to obtain a compound of formula XIII: 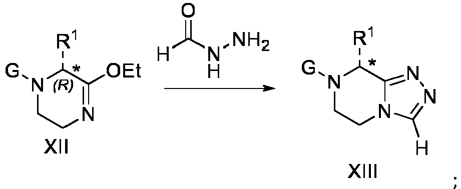
ii) if G = PG, removing G from a compound of formula X to obtain a compound of formula X where G is hydrogen: 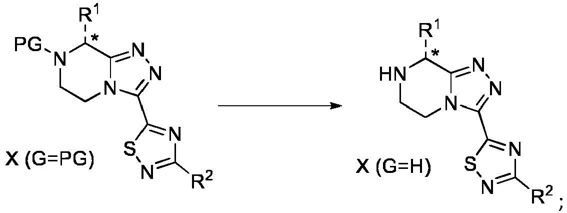
iii) if G = H, transform X into a compound of formula X where G is -CO-Ar¹ :

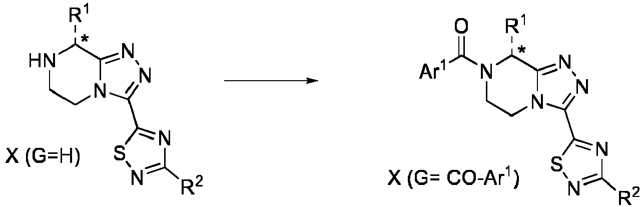

In a further and specific aspect of the invention, compound of formula X produced by the process is Fezolinetant of formula la.

A further aspect of the invention is represented by a compound of formula XV or a salt and/or cocrystal thereof: 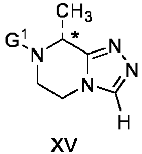 where
- G¹ is: - a 4-fluorobenzoyl group; or - a nitrogen protecting group chosen between optionally substituted carbamate groups (including tert-butoxycarbonyl and benzyloxycarbonyl groups), benzyl and 2,4-dimethoxybenzyl;
- *---: represents one enantiomer R or S of compounds of formula XV or a mixture of the two enantiomers in any proportion including the racemate.

A further specific aspect of the invention is represented by a complex between compound of formula XV and oxalic acid in any proportion.

### Description of the drawings

- **Fig. 1:**: XRPD trace of Compound between oxalic acid and (R)-7-(2,4-dimethoxybenzyl)-8-methyl-5,6,7,8-tetrahydro-[1,2,4]triazolo[4,3-a]pyrazine.
- **Fig. 2:**: DSC trace of Compound between oxalic acid and (R)-7-(2,4-dimethoxybenzyl)-8-methyl-5,6,7,8-tetrahydro-[1,2,4]triazolo[4,3-a]pyrazine.
- **Fig. 3:**: ¹H-NMR spectrum of Compound between oxalic acid and (R)-7-(2,4-dimethoxybenzyl)-8-methyl-5,6,7,8-tetrahydro-[1,2,4]triazolo[4,3-a]pyrazine (Example 19).
- **Fig. 4:**: ¹H-NMR spectrum of (R)-7-(2,4-dimethoxybenzyl)-8-methyl-5,6,7,8-tetrahydro-[1,2,4]triazolo[4,3-a]pyrazine (Example 16).
- **Fig. 5:**: ¹H-NMR spectrum of Benzyl (R)-8-methyl-5,6-dihydro-[1,2,4]triazolo[4,3-a]pyrazine-7(8H)-carboxylate (Example 26).

### Detailed description of the invention

All terms used in this application, unless otherwise specified, are to be understood in their ordinary meaning as known in the relevant technical field.

The term "about" includes the range of experimental errors, which can normally occur performing a measurement, e.g., ± 5% or t 2% or t 1%. Sometimes, such a range can lie within the experimental error, typical of standard methods used for the measurement and/or determination of a given value or range.

The term "room temperature" as used herein refers to a temperature in the range from about 18 °C to about 25 °C.

Unless otherwise indicated, in the context of the present invention the percentage and amount of a certain component in a composition are to be referred to the weight of said component with respect to the total weight of the composition.

Unless otherwise indicated, in the context of the present invention a range of values indicated for a certain parameter, for example the weight or the volume of a component in a mixture, includes the upper and the lower limits of the range, e.g., if the content in weight, or in volume, of a component A in a mixture is indicated as "X to Y" or "between X and Y", the content of A can be X, Y or any of the intermediate values.

Unless otherwise indicated, the data relative to the peaks in the XRPD pattern are related to a diffractogram collected with the Kα radiation of copper (λ =1.5419 Å) at room temperature, and are meant within the common uncertainty due to the instrument measurement, typically ± 0.2 degrees 2θ.

The compounds obtained by the chemical transformations of the present invention can be used without further purification or can be separated and purified by employing conventional methods well known to those skilled in the art, such as crystallization, column chromatography, or by transforming them into a salt or into a co-crystal with an appropriate co-former, or by washing with an organic solvent or with an aqueous solution, optionally adjusting pH.

It will be understood that any one of the compounds described herein also refer to salts or co-crystals thereof.

According to the first aspect, the present invention relates to a process for the preparation of compound of formula X: 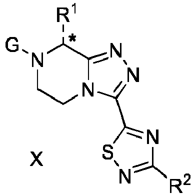 where
- G is: - a -X-Ar¹ group, where X is a carbonyl group CO and Ar¹ is an optionally substituted 5- or 6-membered aryl or heteroaryl group, or - a nitrogen protecting group PG; the nitrogen protecting group PG can be selected from the group including, but not limited to, optionally substituted carbamate groups, optionally substituted alkyl groups, optionally substituted benzyl groups, optionally substituted amido groups, or - a hydrogen;
- R¹ is: an optionally substituted C₁-C₆ alkyl;
- R² is: an optionally substituted C₁-C₆ alkyl; or optionally substituted phenyl group; and
- *---: represents one enantiomer R or S of compounds of formula X or a mixture of the two enantiomers in any proportion including the racemate;
the process including the following step: 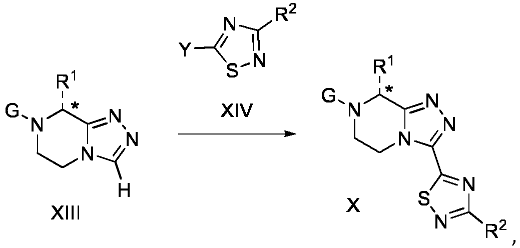 reacting a compound of formula XIII with a compound of formula XIV in a solvent, or a mixture of solvents, in the presence of one or more metal-based catalysts, optionally in the presence of one or more ligands and/or one or more bases, to obtain a compound of formula X, where Y is a leaving group. The leaving group can be an halogen (Cl, Br or I), a sulfonate group (e.g. CH₃SO₂-O-, pTolSO₂-O-, CF₃SO₂-O- or other C₁-C₈ alkyl- or C₆-C₁₀ aryl-sulfonates), a sulfone group (e.g. CH₃SO₂-, pTolSO₂-, CF₃SO₂- or other C₁-C₈ alkyl- or C₆-C₁₀ aryl -sulfones), a sulfoxide group (e.g. CHsSO-, pToISO-, CF₃SO- or other C₁-C₈ alkyl- or C₆-C₁₀ aryl-sulfoxides), a sulfinate group (e.g. CH₃SO-O-, pToISO-O-, CFsSO-O- or other C₁-C₈ alkyl- or C₆-C₁₀ aryl-sulfinates), and the other symbols G, R1, R2 and " *--- " have the same meaning as described above in the first aspect.

In a specific aspect of the invention, *--- represents enantiomer R of compound of formula XII either pure or as major component of the mixture of enantiomers.

In a specific aspect of the invention, the metal in said metal-based catalysts can be palladium, copper, rhodium, nickel, ruthenium, iridium, iron, manganese or other transition metals.

In a preferred aspect of the invention, the metal in said metal-based catalysts can be palladium, or copper, or a mixture thereof. In an even more preferred aspect, such catalysts may be Pd(OAc)₂, Pd(PPh₃)₄, Pd(PPh₃)₂Cl₂, Pd(dppf)Cl₂, PdCl₂, Cul, CuBr, CuCI, Cul₂, CuBr₂, CuCl₂, Cu(OAc)₂, CuO, Cu₂O or a combination thereof.

In a preferred aspect of the invention, such metal-based catalysts may be used in 0.05% to 50% molar ratio with respect to the starting compound of formula XIII.

In another specific aspect of the invention, the ligands can be substituted phosphines or other compounds of phosphorous, such as (but not limited to) triphenylphosphine, tri-tolylphosphine, tributylphosphine, tricyclohexylphosphine, 2,2'-Bis(diphenylphosphino)-1,1'-binaphthyl (*BINAP*), 4,5-Bis(diphenylphosphino)-9,9-dimethylxanthene (Xantphos), 1,1'-bis(diphenylphosphino)ferrocene (*dppf*), bis(diphenylphosphino)ethane (*dppe*), bis[(2-diphenylphosphino)phenyl] ether (*DPEphos*), 1,2-bis-2,5-dimethylphospholane *(Me-DuPhos),* 1,2-bis(2,5-diphenylphospholano)ethane (*Ph-BPE*) dicyclohexyl(2',6'-diisopropoxy-[1,1'-biphenyl]-2-yl)phosphine (*RUphos*); or ligands based on carbon-carbon double bonds or aromatic rings, such as ethylene, propene, cyclooctadiene, cyclooctatetraene, cyclopentadiene, pentamethylcyclopentadiene, benzene, toluene, mesitylene, *p*-cymene, hexamethylbenzene, 2,5-norbornadiene, 1,5-hexadiene; or ligands based on pyridine moieties (*e.g.* 2,2'-bispyridine, 2-(2-pyridinyl)quinoline, 1,10-phenantroline), ethylenediamine moieties (*e.g.* ethylenediamine, N,N'-dimethylethylenediamine, 1,2-diphenylethlenediamine, N,N'-dicyclohexylethylenediamine), nitrile moieties (e.g. benzonitrile, acetonitrile), dione moieties (*e.g.* optionally substituted acetylacetones), ether moieties (*e.g.* ethylene glycol dimethylether, diethylene glycol dimethylether) or N-heterocyclic carbenes (*e.g.* deriving from imidazolium, triazolium, pyrazolium, oxazolium salts), or optionally substituted porphyrin systems. The ligands can be added to the reaction mixture separated from the source of the metal catalyst, or the complex between the metal and the ligands can be preformed and/or isolated as a separated compound which is added to the reaction mixture.

In a preferred aspect of the invention, the ligands are phosphine compounds; in an even more preferred aspect, the ligand is triphenylphosphine, tri-tolylphosphine, tributylphosphine, or tricyclohexylphosphine.

In a preferred aspect of the invention, the ligand may be used in 5% to 70% molar ratio with respect to the starting compound of formula XIII.

In another specific aspect of the invention, the bases can be inorganic, such as (but not limited to) potassium, sodium or cesium carbonate, sodium or potassium bicarbonate, sodium, potassium or cesium hydroxide, sodium or potassium phosphates or hydrogenophosphates; or organic, such as trialkylamines, n-methylimidazole, optionally substituted pyridines, 1,5-diazabiciclo(5.4.0)undec-7-ene (*DBU*), 1,5-Diazabicyclo 4.3.0 non-5-ene (*DBN*), 1,4-diazabicyclo[2.2.2]octane (*DABCO*), phosphazenes.

In a preferred aspect of the invention, the base is potassium carbonate, sodium carbonate or cesium carbonate.

In another specific aspect of the invention, suitable solvents include, but are not limited to toluene, benzene, dichloromethane, xylenes, anisole, chlorobenzene, hexane, heptane, cyclohexane, methylcyclohexane, dioxane, tetrahydrofuran, methyltetrahydrofuran, methyl-tert-butylether, cyclopentylmethylether, C₁-C₄ alcohols, C₁-C₄-alkyl acetates, dimethylformamide, dimethylacetamide, dimethylsulfoxide, water and any mixture thereof, either mono- or bi-phasic.

In a preferred aspect of the invention, the solvent is toluene, or dichloromethane, or xylene, or tetrahydrofuran, or methyltetrahydrofuran, or C₁-C₄ alcohols, or C₁-C₄-alkyl acetates, or water, or any mixture thereof, either mono- or bi-phasic.

In a specific aspect of the invention, G is dimethoxybenzyl (*DMB*), *t*-butoxycarbonyl (*Boc*), benzyloxycarbonyl (*Cbz*) or *p*-fluorobenzoyl.

In another specific aspect of the invention, R¹ is methyl. In another specific aspect of the invention, R² is methyl.

In another specific aspect of the invention, *--- represent enantiomer R of compound of formula X either pure or as major component of the mixture of enantiomers.

In a preferred aspect of the invention, compound of formula X is Fezolinetant: (4-fluorophenyl)-[(8R)-8-methyl-3-(3-methyl-1,2,4-thiadiazol-5-yl)-6,8-dihydro-5H-[1,2,4]triazolo[4,3-a]pyrazin-7-yl]methanone.

Several suitable compounds of formula XIV are known in the literature or can be prepared by variations and/or combinations of known literature procedures.

Another aspect of the invention relates to a process for preparing compound of formula X described in the first aspect which also includes a step to prepare a compound of formula XIII by reacting a compound of formula XII with formohydrazide: 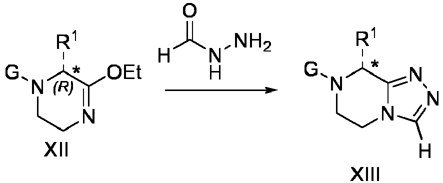 where the symbols G, R1, R2 and " *--- " have the same meaning as described above.

In a specific aspect of the invention, the reaction in this step may be carried out in a solvent medium which includes, but is not limited to, C₁-C₄ alcohols, tetrahydrofuran, methyltetrahydrofuran, methyl-tert-butylether, cyclopentylmethylether, dichloromethane, toluene, benzene, xylenes, anisole, chlorobenzene, hexane, heptane, cyclohexane, methylcyclohexane, dioxane, dimethylformamide, dimethylacetamide, dimethylsulfoxide, melted formohydrazide, C₁-C₄-alkyl acetates and any mixture thereof, either mono- or bi-phasic.

Compounds of formula XII can be prepared according to procedures reported in the literature, *e.g.* in patents EP 2,552,920 B1, EP 2,948,455 B1, EP 2,763,992 B9, EP 3,197,876 B1.

Another aspect of the invention relates to the process for preparing compound of formula X described in the first aspect which also includes a step to prepare compound of formula X where G is hydrogen starting from a compound of formula X where G = PG: 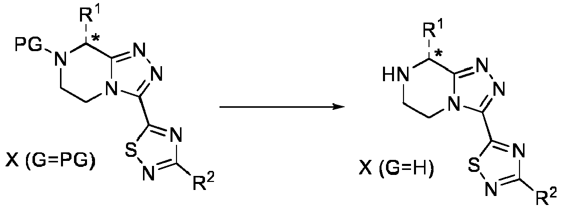 where the symbols PG, R1, R2 and " *---" have the same meaning as described above.

The conditions of this step depend on the nature of PG; for instance, if PG = Boc or DMB, it can be removed by a reaction involving an acidic treatment; if PG = Cbz it can be removed by a reaction involving a basic hydrolysis or a catalytic hydrogenation. A person skilled in the art can envisage other methods to remove a nitrogen-protecting group according to the known literature.

Another aspect of the invention relates to the process for preparing compound of formula X described in the first aspect which also includes a step to prepare compound of formula X where G is -CO-Ar¹ starting from a compound of formula X where G = H: 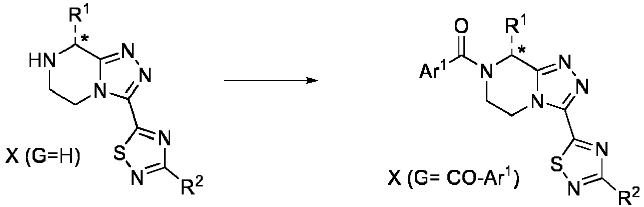 where the symbols Ar¹, R1, R2 and " *---" have the same meaning as described above.

This reaction is accomplished by reacting a compound of formula X where G = H with an activated form of a carboxylic acid Ar¹COOH, either prepared in a separated step or in the same reaction mixture; such activated forms for the synthesis of amides are known in the art and include for instance (but are not limited to) acyl chlorides, acyl bromides, acyl fluorides, activated esters, imidazolides, mixed anhydrides or acyl-isoureas. In a specific aspect of this invention, the activated form is the acyl chloride Ar¹COCl. In a preferred aspect of the invention, Ar¹ is *p*-fluorophenyl. In another preferred aspect of the invention, compound of formula X is Fezolinetant.

A further aspect of the invention is represented by a compound of formula XV: 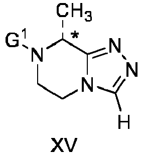 where
- G¹ is: - a 4-fluorobenzoyl group; or - a nitrogen protecting group chosen between optionally substituted carbamate groups (including tert-butoxycarbonyl and benzyloxycarbonyl groups), benzyl and 2,4-dimethoxybenzyl;
- *---: represents one enantiomer R or S of compounds of formula XV or a mixture of the two enantiomers in any proportion including the racemate.

In a specific aspect of the invention, *--- in compounds of formula XV represent enantiomer R of compound of formula XV either pure or as major component of the mixture of enantiomers.

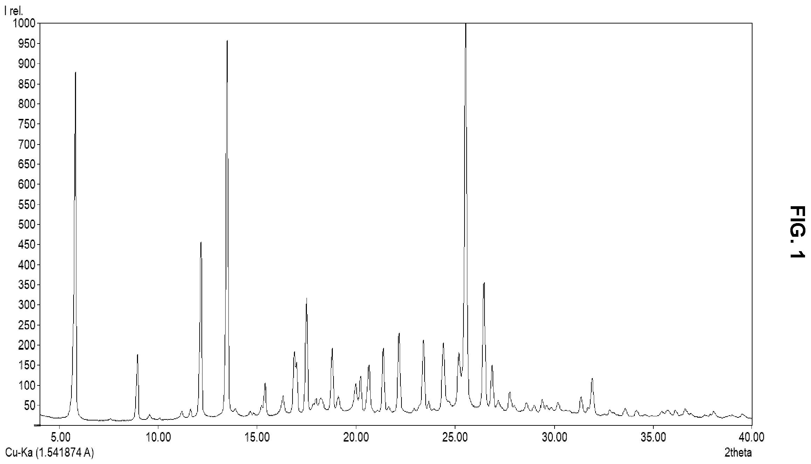

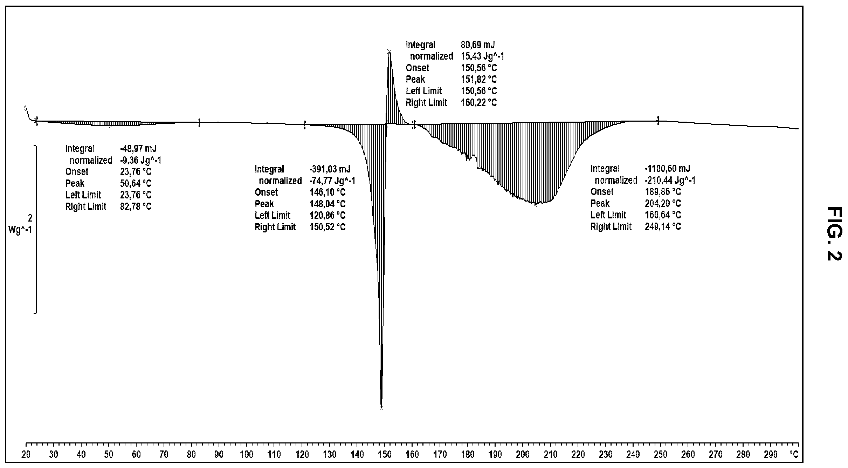

A further specific aspect of the invention is represented by a complex between compound of formula XV and oxalic acid in any proportion, conveniently used to isolate compound of formula XV in a purified form. A preferred aspect of the invention is represented by a complex between oxalic acid and compound of formula XV where G¹ is 2,4-dimethoxybenzyl and "*--" represent the R enantiomer as major isomer; a further preferred aspect of the invention is represented by such complex in crystalline form; a further preferred aspect of the invention is represented by a crystal form of such complex characterized by having a powder X-ray diffractogram comprising peaks at 5.8, 9.0, 12.2, 13.5, 25.6 and 26.5 ± 0.2 degrees 2-Theta, when measured at room temperature with Cu-Kα radiation having a wavelength of 1.5419 Å; more preferably, the powder X-ray diffractogram of such crystal form also comprises one peak at least one of the following positions: 15.4, 17.0, 17.5, 18.8, 20.3, 20.7, 21.4, 22.2, 23.4, 24.4, 25.2, 26.9, 27.8, 29.4, 31.4 or 31.9 ± 0.2 degrees 2-Theta; even more preferably, the diffractogram is essentially correspondent to the one reported in Fig. 1; in an additional aspect, the described complex shows a differential scanning calorimetry trace (DSC; measured at a heating rate of 10 °C/min) essentially correspondent to the one reported in Fig. 2.

### Experimental examples

### a) Synthesis of compounds of formula X from compounds of formula XIII

### Example 1

### (R)-5-(7-(2,4-dimethoxybenzyl)-8-methyl-5,6,7,8-tetrahydro-[1,2,4]triazolo[4,3-a]pyrazin-3-yl)-3-methyl-1,2,4-thiadiazole

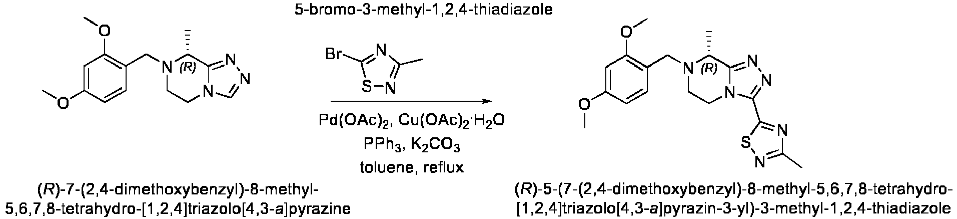

To a solution of (R)-7-(2,4-dimethoxybenzyl)-8-methyl-5,6,7,8-tetrahydro-[1,2,4]triazolo[4,3-a]pyrazine (780 mg; 2.71 mmol, 1 eq) in toluene (11.7 mL), was added 5-bromo-3-methyl-1,2,4-thiadiazole (630 mg; 3.52 mmol; 1.3 eq) under N₂ atmosphere. Then Pd(OAc)₂ (18.2 mg; 0.081 mmol, 0.03 eq), Cu(OAc)₂·H₂O (107.8 mg; 0.542 mmol, 0.2 eq), PPhs (354.8 mg; 1.35 mmol, 0.5 eq) and K₂CO₃ (747.5 mg; 5.42 mmol, 2 eq) were sequentially added. The reaction mixture was stirred at reflux temperature for 16 h, then it was allowed to cool to room temperature. H₂O (25 mL) and celite were added, the mixture was filtered under vacuum on a pad of celite, the phases were separated and the aqueous layer was back-extracted with toluene (2 × 15 mL); the combined organic layers were dried over Na₂SO₄, filtered and evaporated to dryness. The crude product was purified by flash chromatography, eluting with EtOAc:Hexane, from 50:50 to 90:10, yielding 521 mg (y = 51%) of the title compound as a pale-yellow solid.
LC-MS: *m*/*z* = 387.4 (M+H)⁺
¹H NMR (500 MHz, MeOD): δ (ppm): 7.29 (d, *J* = 8.3 Hz, 1H), 6.57 (d, *J* = 2.4 Hz, 1H), 6.54 (dd, *J* = 8.3, 2.4 Hz, 1H), 4.57 (dt, *J* = 13.3, 4.7 Hz, 1H), 4.33 (ddd, *J* = 13.0, 8.0, 4.6 Hz, 1H), 4.08 (q, *J* = 6.7 Hz, 1H), 3.98 (d, *J* = 13.4 Hz, 1H), 3.83 (s, 3H), 3.81 (s, 3H), 3.67 (d, *J* = 13.4 Hz, 1H), 3.37-3.33 (m, 1H), 2.89 - 2.81 (m, 1H), 2.72 (s, 3H), 1.71 (d, *J* = 6.7 Hz, 3H).

¹³C NMR (126 MHz, MeOD): δ (ppm): 174.24, 174.10, 160.80, 159.02, 156.61, 145.41, 131.33, 117.10, 104.28, 97.95, 54.47, 54.38, 52.99, 49.78, 44.83, 44.11, 17.31, 16.39.

### Example 2

(R)-5-(7-(2,4-dimethoxybenzyl)-8-methyl-5,6,7,8-tetrahydro-[1,2,4]triazolo[4,3-a]pyrazin-3-yl)-3-methyl-1,2,4-thiadiazole

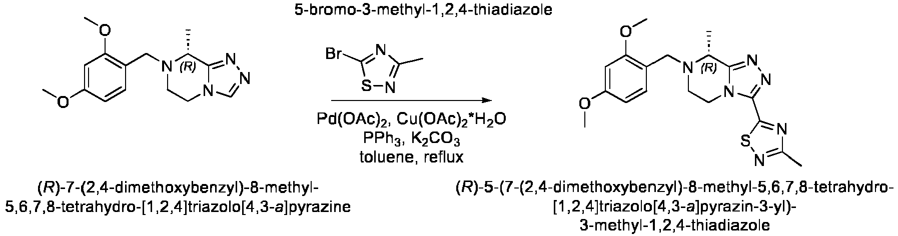

To a solution of (R)-7-(2,4-dimethoxybenzyl)-8-methyl-5,6,7,8-tetrahydro-[1,2,4]triazolo[4,3-a]pyrazine (15.10 g; 52.43 mmol, 1 eq) in toluene (181.2 mL), was added 5-bromo-3-methyl-1,2,4-thiadiazole (12.20 g; 68.16 mmol; 1.3 eq) under N₂ atmosphere. Then Pd(OAc)₂ (529.3 mg; 2.36 mmol, 0.045 eq), Cu(OAc)₂·H₂O (3.13 g; 15.73 mmol; 0.3 eq), PPh₃ (10.30 g; 39.32 mmol; 0.75 eq) and K₂CO₃ (14.47 g; 104.86 mmol, 2 eq) were sequentially added. The reaction mixture was stirred at reflux temperature for 16 h, then it was allowed to cool to room temperature and H₂O (350 mL) and celite were added. The mixture was filtered under vacuum on a pad of celite, the phases were separated and the aqueous layer was back-extracted with toluene (3 × 100 mL), the combined organic layers were dried over Na₂SO₄, filtered and evaporated to dryness. The resulting crude product was purified by flash chromatographic following the conditions described in Example 1, yielding 9.5 g (y = 47%) of the title compound as a pale-yellow solid.

¹H-NMR and LC-MS matched those reported in Example 1.

### Example 3

(R)-5-(7-(2,4-dimethoxybenzyl)-8-methyl-5,6,7,8-tetrahydro-[1,2,4]triazolo[4,3-a]pyrazin-3-yl)-3-methyl-1,2,4-thiadiazole

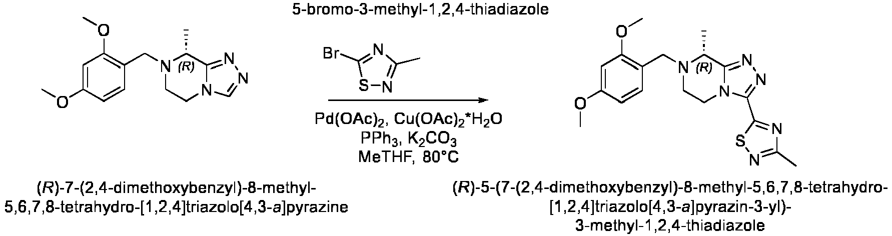

To a solution of (R)-7-(2,4-dimethoxybenzyl)-8-methyl-5,6,7,8-tetrahydro-[1,2,4]triazolo[4,3-a]pyrazine (0.8 g, 2.77 mmol, 1 eq) in MeTHF (9.6 mL) under N₂, was added 5-bromo-3-methyl-1,2,4-thiadiazole (0.65 g, 3.61 mmol, 1.3 eq); then Pd(OAc)₂ (28 mg, 0.12 mmol, 0.045 eq), Cu(OAc)₂·H₂O (0.165 g, 0.831 mmol, 0.3 eq), PPhs (0.544 g, 2.08 mmol, 0.75 eq) and K₂CO₃ (0.765 g, 5.54 mmol, 2 eq) were sequentially added. The reaction was stirred at 80°C overnight and then it was allowed to cool to room temperature. Celite was added to the reaction mixture, which was then filtered; the filtrate was washed with water (15 mL), the organic phase was collected, dried over Na₂SO₄, filtered and concentrated to dryness. The resulting crude compound was purified by flash chromatography following the conditions described in Example 1, yielding 198 mg (y = 18%) of the title compound as a pale-yellow solid.

¹H-NMR and LC-MS matched those reported in Example 1.

### Example 4

(R)-5-(7-(2,4-dimethoxybenzyl)-8-methyl-5,6,7,8-tetrahydro-[1,2,4]triazolo[4,3-a]pyrazin-3-yl)-3-methyl-1,2,4-thiadiazole

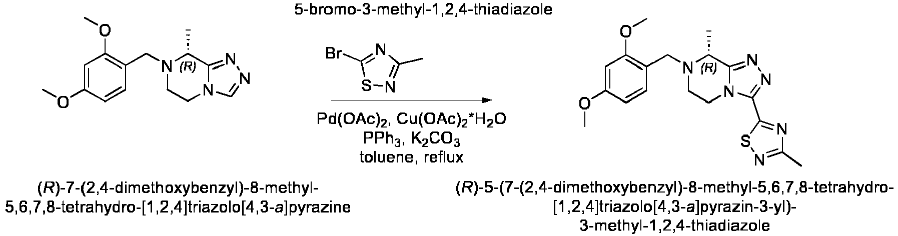

To a solution of (R)-7-(2,4-dimethoxybenzyl)-8-methyl-5,6,7,8-tetrahydro-[1,2,4]triazolo[4,3-a]pyrazine (1.02 g; 3.54 mmol, 1 eq) in toluene (12 mL), was added 5-bromo-3-methyl-1,2,4-thiadiazole (1.20 g; 6.72 mmol; 1.9 eq) under N₂ atmosphere. Then, were sequentially introduced Pd(OAc)₂ (11.91 mg; 0.053 mmol, 0.015 eq), Cu(OAc)₂·H₂O (282 mg; 1.42 mmol; 0.4 eq), PPhs (927 mg; 3.54 mmol; 1 eq) and K₂CO₃ (977 mg; 7.08 mmol, 2 eq). The reaction mixture was refluxed for 16 h, then it was allowed to cool to room temperature and H₂O (30 mL) and celite were added. The mixture was filtered under vacuum, and the resulting two phases were separated. The aqueous layer was back-extracted with toluene (20 mL) and the combined organic layers were dried over Na₂SO₄, filtered and evaporated to dryness. The resulting crude compound was purified by flash chromatography following the conditions described in Example 1, yielding 1.03 g (y = 75%) of the title compound as a pale-yellow solid.

¹H-NMR and LC-MS matched those reported in Example 1.

### Example 5

(R)-5-(7-(2,4-dimethoxybenzyl)-8-methyl-5,6,7,8-tetrahydro-[1,2,4]triazolo[4,3-a]pyrazin-3-yl)-3-methyl-1,2,4-thiadiazole

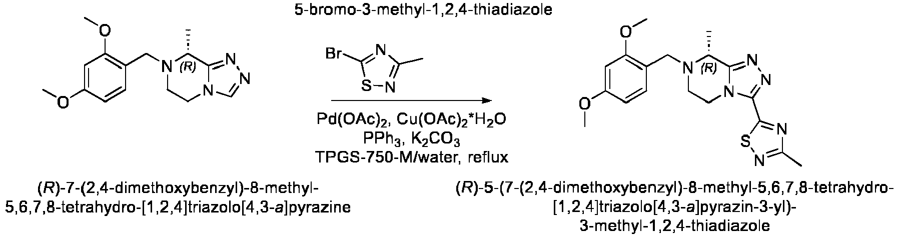

To a solution of (R)-7-(2,4-dimethoxybenzyl)-8-methyl-5,6,7,8-tetrahydro-[1,2,4]triazolo[4,3-a]pyrazine (660 mg; 2.29 mmol, 1 eq) in 5% wt TPGS-750-M/H₂O (9.2 mL), was added 5-bromo-3-methyl-1,2,4-thiadiazole (532.7 mg; 2.97 mmol; 1.3 eq) under N₂ atmosphere. Then, were sequentially introduced Pd(OAc)₂ (23.1 mg; 0.103 mmol, 0.045 eq), Cu(OAc)₂·H₂O (137.1 mg; 0.69 mmol; 0.3 eq), PPhs (450.3 mg; 1.72 mmol; 0.75 eq) and K₂CO₃ (632.7 mg; 4.58 mmol, 2 eq) and the resulting reaction mixture was refluxed for 20 h. Then it was allowed to cool to room temperature and EtOAc (9 mL) and celite were added. The mixture was filtered under vacuum, and the resulting two phases were separated. The aqueous layer was extracted with EtOAc (2 × 10 mL) and the combined organic layers were dried over Na₂SO₄, filtered and evaporated to dryness. The resulting crude compound was purified by flash chromatography following the conditions described in Example 1, yielding 354 mg (y = 40%) of the title compound as a pale-yellow solid.

¹H-NMR and LC-MS matched those reported in Example 1.

### Example 6

(R)-5-(7-(2,4-dimethoxybenzyl)-8-methyl-5,6,7,8-tetrahydro-[1,2,4]triazolo[4,3-a]pyrazin-3-yl)-3-methyl-1,2,4-thiadiazole

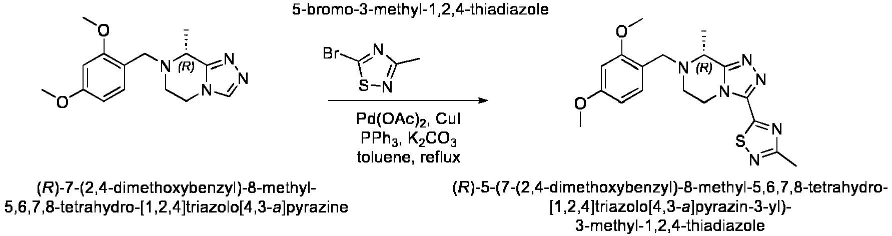

To a solution of (R)-7-(2,4-dimethoxybenzyl)-8-methyl-5,6,7,8-tetrahydro-[1,2,4]triazolo[4,3-a]pyrazine (32.4 g; 112.36 mmol, 1 eq) in toluene (250 mL), was added a solution of 5-bromo-3-methyl-1,2,4-thiadiazole (30.18 g; 168.55 mmol, 1.5 eq) in 120 mL of toluene under N₂ atmosphere. Then, were sequentially introduced Pd(OAc)₂ (0.252 g; 1.12 mmol, 0.01 eq), Cul (4.28 g; 22.47 mmol; 0.2 eq), PPh₃ (14.74 g; 56.18 mmol; 0.5 eq) and K₂CO₃ (31.06 g; 224.72 mmol, 2 eq) and the resulting reaction mixture was heated to reflux for 16 h. The mixture was allowed to cool to room temperature, then H₂O (730 mL) and celite (20 g) were added; after stirring for about 90 minutes the mixture was filtered on a pad of celite, washing with 150 mL of toluene. The layers were separated and the aqueous layer was back-extracted with toluene (250 mL); the combined organic layers were cooled to 0°C, then 3N HCl (250 mL) was added; the biphasic mixture was stirred while allowing it to warm up to room temperature and the two phases were separated. The aqueous layer was collected, cooled to 0°C and brought to basic pH with 100 mL of 28% NH₄OH solution. The biphasic mixture was stirred while allowing it to warm up to room temperature, then the organic layer was collected and evaporated to dryness under reduced pressure. 41.31 g (y = 95.1%) of the title compound as a pale-brown solid were obtained.

The crude product was of sufficient purity to be used in the next step without further purification; an analytical sample was obtained after chromatographic purification according to the conditions described in Example 1. ¹H-NMR and LC-MS matched those reported in Example 1.

### Example 7

(R)-5-(7-(2,4-dimethoxybenzyl)-8-methyl-5,6,7,8-tetrahydro-[1,2,4]triazolo[4,3-a]pyrazin-3-yl)-3-methyl-1,2,4-thiadiazole

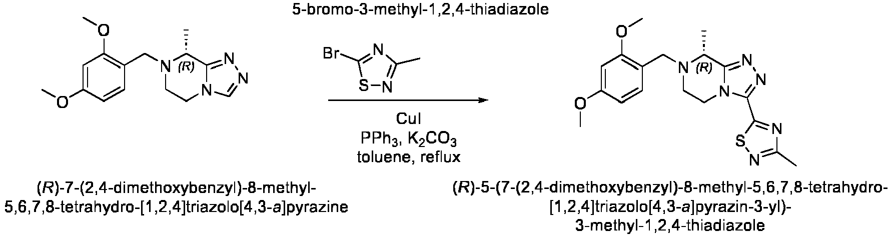

To a solution of (R)-7-(2,4-dimethoxybenzyl)-8-methyl-5,6,7,8-tetrahydro-[1,2,4]triazolo[4,3-a]pyrazine) (580 mg; 2.01 mmol, 1 eq) in toluene (7 mL), was added 5-bromo-3-methyl-1,2,4-thiadiazole (720.3 mg; 4.02 mmol; 2 eq) under N₂ atmosphere. To this reaction mixture were sequentially added Cul (76.6 mg; 0.40 mmol; 0.2 eq), PPhs (263.8 mg; 1.01 mmol; 0.5 eq) and K₂CO₃ (556.0 mg; 4.02 mmol, 2 eq) and the resulting reaction mixture was refluxed for 22 h, then it was allowed to cool to room temperature. Water (17 mL) and celite were added and the mixture was filtered under vacuum washing with 10 mL of toluene. The organic layer was collected, dried over Na₂SO₄, filtered and evaporated to dryness. The resulting crude compound was purified by flash chromatography following the conditions described in Example 1, yielding 380 mg (y = 49%) of the title compound as a pale-yellow solid.

¹H-NMR and LC-MS matched those reported in Example 1.

### Example 8

(R)-5-(7-(2,4-dimethoxybenzyl)-8-methyl-5,6,7,8-tetrahydro-[1,2,4]triazolo[4,3-a]pyrazin-3-yl)-3-methyl-1,2,4-thiadiazole

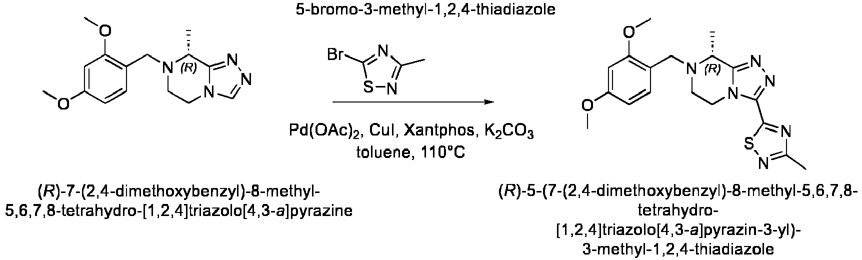

To a solution of (R)-7-(2,4-dimethoxybenzyl)-8-methyl-5,6,7,8-tetrahydro-[1,2,4]triazolo[4,3-a]pyrazine (480 mg; 1.66 mmol, 1 eq) in toluene (5.8 mL), was added 5-bromo-3-methyl-1,2,4-thiadiazole (520 mg; 2.90 mmol; 1.7 eq) under N₂ atmosphere. To this reaction mixture were added Pd(OAc)₂ (38 mg; 0.17 mmol, 0.1 eq) Cul (66 mg; 0.35 mmol; 0.2 eq), 4,5-Bis(diphenylphosphino)-9,9-dimethylxanthene (xantphos) (188 mg; 0.32 mmol; 0.2 eq) and K₂CO₃ (492 mg; 3.56 mmol, 2.1 eq) and the resulting reaction mixture was refluxed for 22 h, then it was allowed to cool to room temperature and H₂O (10 mL) and celite were added. The mixture was filtered under vacuum, washing with toluene, and the resulting two phases were separated. The organic phase was washed with 20 mL of HCl 3M, then it was discarded. The acidic aqueous phase was basified to pH 10 with 8 mL of NH₄OH (28% aqueous solution) and finally extracted with 20 mL of DCM. The organic layer was dried over Na₂SO₄, filtered and evaporated to dryness. The resulting crude compound was purified by flash chromatography following the conditions described in Example 1, yielding 289 mg (y = 45%) of the title compound as a pale-yellow solid.

¹H-NMR and LC-MS matched those reported in Example 1.

### Example 9

(R)-5-(7-(2,4-dimethoxybenzyl)-8-methyl-5,6,7,8-tetrahydro-[1,2,4]triazolo[4,3-a]pyrazin-3-yl)-3-methyl-1,2,4-thiadiazole

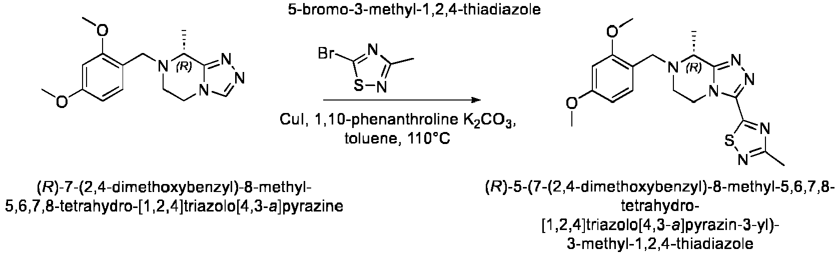

To a solution of (R)-7-(2,4-dimethoxybenzyl)-8-methyl-5,6,7,8-tetrahydro-[1,2,4]triazolo[4,3-a]pyrazine (431 mg; 1.49 mmol, 1 eq) in toluene (5 mL), was added 5-bromo-3-methyl-1,2,4-thiadiazole (450 mg; 2.51 mmol; 1.7 eq) under N₂ atmosphere. To this reaction mixture were added Cul (14 mg; 0.07 mmol; 0.05 eq), 1,10-phenanthroline (27 mg; 0.15 mmol; 0.1 eq) and K₂CO₃ (414 mg; 3.00 mmol, 2 eq) and the resulting reaction mixture was refluxed for 25 h. It was then allowed to cool to room temperature and H₂O (13 mL) and celite were added. The mixture was filtered under vacuum washing with 9 mL of toluene, and the resulting two phases were separated. The organic layer was dried over Na₂SO₄, filtered and evaporated to dryness. The resulting crude compound was purified by flash chromatography following the conditions described in Example 1, yielding 116 mg (y = 20%) of the title compound as a pale-yellow solid.

¹H-NMR and LC-MS matched those reported in Example 1.

### Example 10

(R)-5-(7-(2,4-dimethoxybenzyl)-8-methyl-5,6,7,8-tetrahydro-[1,2,4]triazolo[4,3-a]pyrazin-3-yl)-3-methyl-1,2,4-thiadiazole

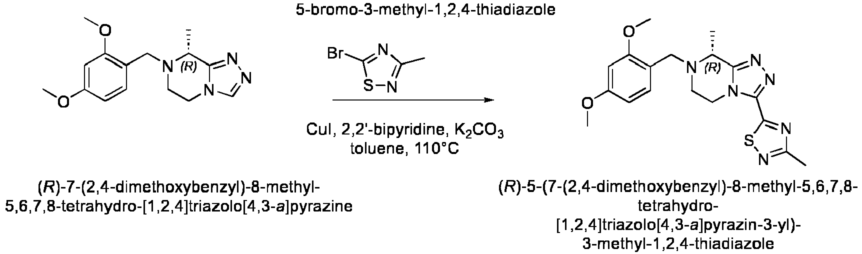

To a solution of (R)-7-(2,4-dimethoxybenzyl)-8-methyl-5,6,7,8-tetrahydro-[1,2,4]triazolo[4,3-a]pyrazine (589 mg; 2.04 mmol, 1 eq) in toluene (7 mL), was added 5-bromo-3-methyl-1,2,4-thiadiazole (550 mg; 3.07 mmol; 1.5 eq) under N₂ atmosphere. To this reaction mixture were added Cul (20 mg; 0.11 mmol; 0.05 eq), 2,2'-bipyridine (33 mg; 0.21 mmol; 0.1 eq) and K₂CO₃ (570 mg; 4.12 mmol, 2.0 eq) and the resulting reaction mixture was refluxed for 16 h. It was then allowed to cool to room temperature and H₂O (10 mL) and celite were added. The mixture was filtered under vacuum washing with toluene, and the resulting two phases were separated. The organic layer was dried over Na₂SO₄, filtered and evaporated to dryness. The resulting crude compound was purified by flash chromatography following the conditions described in Example 1, yielding 259 mg (y = 33%) of the title compound as a pale-yellow solid.

¹H-NMR and LC-MS matched those reported in Example 1.

### Example 11

(R)-5-(7-(2,4-dimethoxybenzyl)-8-methyl-5,6,7,8-tetrahydro-[1,2,4]triazolo[4,3-a]pyrazin-3-yl)-3-methyl-1,2,4-thiadiazole

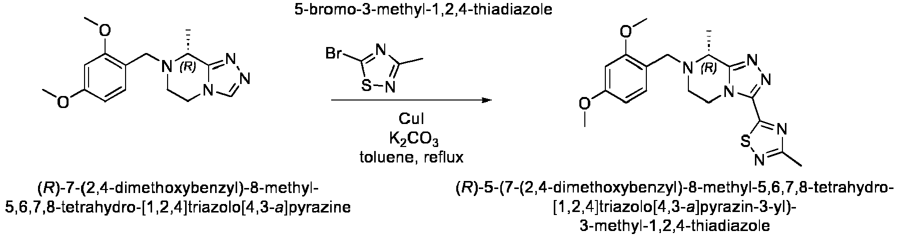

To a solution of (R)-7-(2,4-dimethoxybenzyl)-8-methyl-5,6,7,8-tetrahydro-[1,2,4]triazolo[4,3-a]pyrazine) (348.0 mg; 1.21 mmol, 1 eq) in toluene (4 mL), was added 5-bromo-3-methyl-1,2,4-thiadiazole (433.1 mg; 2.41 mmol; 2 eq) under N₂ atmosphere. To this reaction mixture were added Cul (45.0 mg; 0.24 mmol; 0.2 eq) and K₂CO₃ (333.6 mg; 2.41 mmol, 2 eq) and the resulting reaction mixture was refluxed for 18 h. It was then allowed to cool to room temperature and H₂O (10 mL) and celite were added. The mixture was filtered under vacuum washing with 7 mL of toluene, and the resulting two phases were separated. The organic layer was dried over Na₂SO₄, filtered and evaporated to dryness. The resulting crude compound was purified by flash chromatography following the conditions described in Example 1, yielding 145 mg (y = 31%) of the title compound as a pale-yellow solid.

¹H-NMR and LC-MS matched those reported in Example 1.

### Example 12

(R)-5-(7-(2,4-dimethoxybenzyl)-8-methyl-5,6,7,8-tetrahydro-[1,2,4]triazolo[4,3-a]pyrazin-3-yl)-3-methyl-1,2,4-thiadiazole

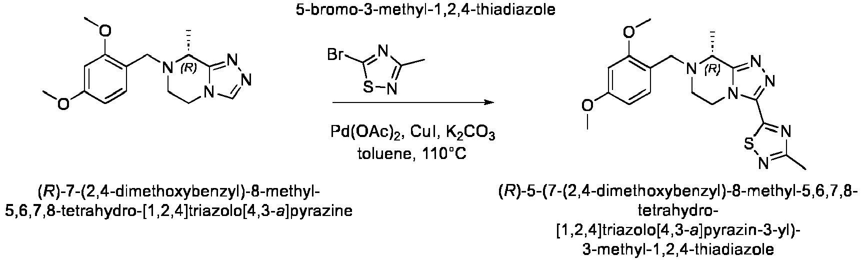

To a solution of (R)-7-(2,4-dimethoxybenzyl)-8-methyl-5,6,7,8-tetrahydro-[1,2,4]triazolo[4,3-a]pyrazine (426 mg; 1.48 mmol, 1 eq) in toluene (5.1 mL), was added 5-bromo-3-methyl-1,2,4-thiadiazole (420 mg; 2.35 mmol; 1.6 eq) under N₂ atmosphere. To this reaction mixture were added Pd(OAc)₂ (17 mg, 0.076 mmol, 0.05 eq), Cul (57 mg; 0.30 mmol; 0.2 eq) and K₂CO₃ (408 mg; 2.95 mmol, 2.0 eq) and the resulting reaction mixture was refluxed for 4 h, then it was allowed to cool to room temperature and H₂O (8.5 mL) was added. The mixture was filtered under vacuum on a celite pad, washing with toluene, and the resulting two phases were separated. The organic layer was dried over Na₂SO₄, filtered and evaporated to dryness. The resulting crude compound was purified by flash chromatography following the conditions described in Example 1, yielding 200 mg (y = 35%) of the title compound as a pale-yellow solid.

¹H-NMR and LC-MS matched those reported in Example 1.

### Example 13

(R)-5-(7-(2,4-dimethoxybenzyl)-8-methyl-5,6,7,8-tetrahydro-[1,2,4]triazolo[4,3-a]pyrazin-3-yl)-3-methyl-1,2,4-thiadiazole

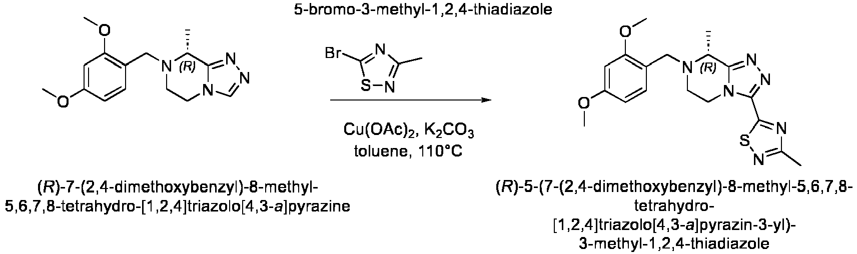

To a solution of (R)-7-(2,4-dimethoxybenzyl)-8-methyl-5,6,7,8-tetrahydro-[1,2,4]triazolo[4,3-a]pyrazine (442 mg; 1.53 mmol, 1 eq) in toluene (5.3 mL), was added 5-bromo-3-methyl-1,2,4-thiadiazole (550 mg; 3.07 mmol; 2.0 eq) under N₂ atmosphere. To this reaction mixture were added Cu(OAc)₂·H₂O (63 mg; 0.32 mmol; 0.2 eq) and K₂CO₃ (423 mg; 3.06 mmol, 2.0 eq) and the resulting reaction mixture was refluxed for 17 h, then it was allowed to cool to room temperature. H₂O (9 mL) was added, the mixture was filtered under vacuum on a celite pad, washing with toluene, and the resulting two phases were separated. The organic layer was dried over Na₂SO₄, filtered and evaporated to dryness. The resulting crude compound was purified by flash chromatography following the conditions described in Example 1, yielding 183 mg (y = 31%) of the title compound as a pale-yellow solid.

¹H-NMR and LC-MS matched those reported in Example 1.

### Example 14

(R)-(4-fluorophenyl)(8-methyl-3-(3-methyl-1,2,4-thiadiazol-5-yl)-5,6-dihydro-[1,2,4] triazolo[4,3-a]pyrazin-7(8H)-yl)methanone

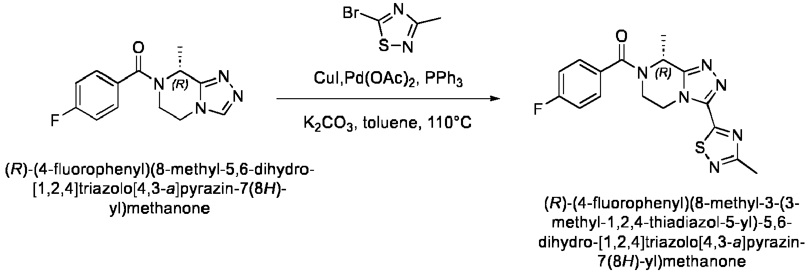

To a suspension of (R)-(4-fluorophenyl)(8-methyl-5,6-dihydro-[1,2,4]triazolo[4,3-a]pyrazin-7(8H)-yl)methanone (139 mg; 0.53 mmol, 1.0 eq) in toluene (2.1 mL), was added 5-bromo-3-methyl-1,2,4-thiadiazole (180 mg; 1.00 mmol; 1.9 eq) under N₂ atmosphere. To this reaction mixture were added Pd(OAc)₂ (14.6 mg; 0.065 mmol, 0.1 eq), Cul (19.6 mg; 0.1 mmol; 0.2 eq), PPhs (71.5 mg; 0.27 mmol; 0.5 eq) and K₂CO₃ (142.5 mg; 1.03 mmol, 1.9 eq) and the resulting reaction mixture was refluxed for 18 h, then it was allowed to cool to room temperature and HCl 4M was added up to pH about 1. The mixture was filtered and the resulting two phases were separated. The acidic aqueous layer was basified with NaOH (pellets) until pH > 12 and extracted with DCM. The organic layer was evaporated to dryness under vacuum, yielding 98 mg (y = 52%) of the title compound as a yellowish powder.

¹H-NMR and LC-MS matched those reported in Example 23 below.

### Example 15

Benzyl (R)-8-methyl-3-(3-methyl-1,2,4-thiadiazol-5-yl)-5,6-dihydro-[1,2,4]triazolo[4,3-a]pyrazine-7(8H)-carboxylate

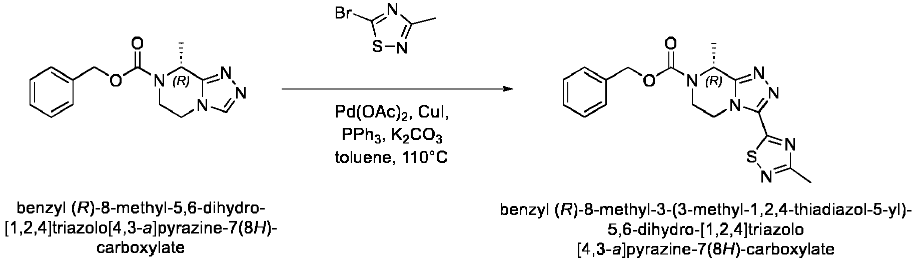

To a solution of benzyl (R)-8-methyl-5,6-dihydro-[1,2,4]triazolo[4,3-a]pyrazine-7(8H)-carboxylate (960 mg; 3.53 mmol, 1 eq) in toluene (12 mL), was added 5-bromo-3-methyl-1,2,4-thiadiazole (820 mg; 4.58 mmol; 1.3 eq) under N₂ atmosphere. To this reaction mixture were added Pd(OAc)₂, (63 mg, 0.282 mmol, 0.08 eq.), Cul (134 mg, 0.706 mmol, 0.2 eq), PPhs (463 mg, 1.77 mmol, 0.5 eq) and K₂CO₃ (976 mg; 7.06 mmol, 2.0 eq) and the resulting reaction mixture was refluxed for 16 h. It was then allowed to cool to room temperature and H₂O (20 mL) was added. The mixture was filtered under vacuum on a celite pad, washing with toluene, and the resulting two phases were separated. The organic layer was dried over Na₂SO₄, filtered and evaporated to dryness. The crude product was purified by flash column chromatography eluting with EtOAc:Cyclohexane (70:30), yielding 0.259 g (y = 20%) of the title compound as an orange powder.
LC-MS: *m*/*z* = 371.3 (M+H)⁺
¹H NMR (500 MHz, MeOD): δ (ppm): 7.45-7.33 (m, 5H), 5.65 (q, *J* = 6.8 Hz, 1H), 5.28-5.21 (m, 2H), 4.92-4.88 (m, 1H), 4.54 (dd, *J* = 14.4, 3.9 Hz, 1H), 4.25-4.19 (m, 1H), 3.54-3.50 (m, 1H), 2.74 (s, 3H), 1.65 (d, *J* = 6.9 Hz, 3H).

### b) Synthesis of compounds of formula XIII from compounds of formula XII

### Example 16

(R)-7-(2,4-dimethoxybenzyl)-8-methyl-5,6,7,8-tetrahydro-[1,2,4]triazolo[4,3-a]pyrazine

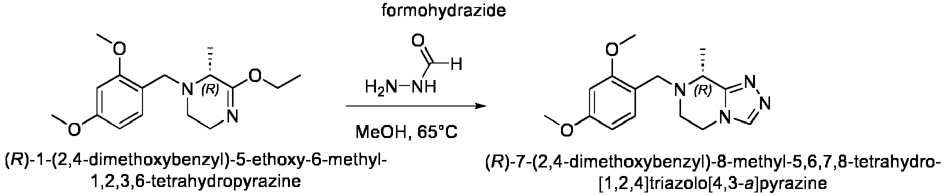

To a suspension of (R)-1-(2,4-dimethoxybenzyl)-5-ethoxy-6-methyl-1,2,3,6-tetrahydropyrazine (16.59 g; 56.75 mmol) in MeOH (34 mL), under N₂ atmosphere, was added formohydrazide (3.58 g; 59.59 mmol, 1.05 eq). The resulting mixture was heated at 65 °C for 3 hours and then it was allowed to cool to room temperature. The solvent was evaporated *in vacuo* and the oily residue was partitioned between CH₂Cl₂ (170 mL) and 1N HCl (170 mL). The phases were separated and the aqueous layer was washed with CH₂Cl₂ (1 × 170 mL) and then brought to pH 11 with 32% NaOH. The aqueous layer was extracted with CH₂Cl₂ (3 × 150 mL) and the combined organic layers were dried over anhydrous Na₂SO₄, filtered and concentrated under reduced pressure. The crude product was purified by flash column chromatography, using Isolera One^{®} Biotage system, eluting with EtOAc/MeOH (gradient from 0% to 10% of MeOH; flow 50 mL/min), yielding 11.40 g (y = 70%) of the title compound as a colorless dense oil.

### LC-MS: m/z = 289.3 (M+H)⁺

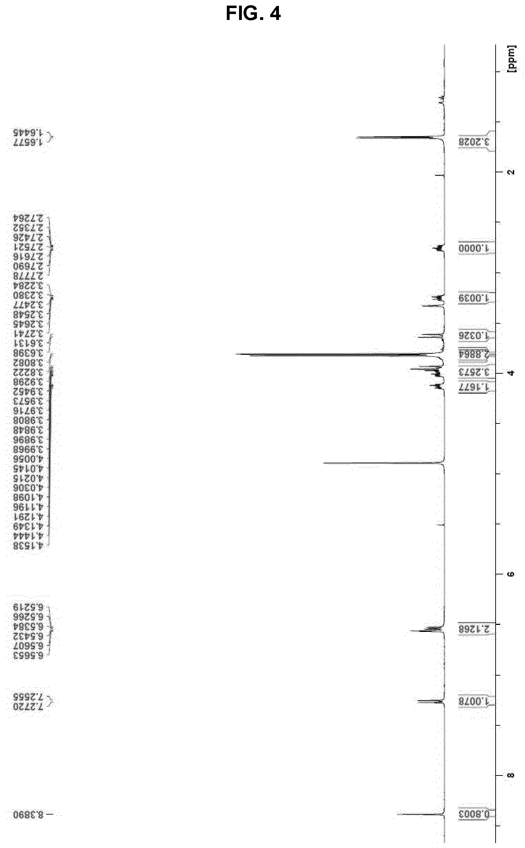

¹H NMR (500 MHz, MeOD): δ (ppm): 8.37 (s, 1H), 7.25 (d, *J* = 8.3 Hz, 1H), 6.56 (d, *J* = 2.3 Hz, 1H), 6.53 (dd, *J* = 8.3, 2.4 Hz, 1H), 4.11 (dt, *J* = 12.5, 4.8 Hz, 1H), 3.97 (m, 3H), 3.82 (s, 3H), 3.81 (s, 3H), 3.61 (d, *J* = 13.4 Hz, 1H), 3.23 (dt, *J* = 13.2, 4.8 Hz, 1H), 2.73 (ddd, *J* = 12.9, 8.1, 4.4 Hz, 1H), 1.63 (d, *J* = 6.6 Hz, 3H) - reported in Fig. 4 ¹³C NMR (126 MHz, MeOD): δ (ppm): 160.77, 159.02, 153.84, 142.32, 131.33, 117.25, 104.28, 97.95, 54.47, 54.39, 52.87, 49.84, 44.93, 41.69, 16.39.

### Example 17

(R)-7-(2,4-dimethoxybenzyl)-8-methyl-5,6,7,8-tetrahydro-[1,2,4]triazolo[4,3-a]pyrazine

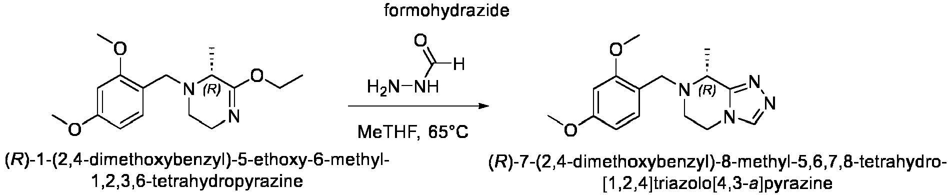

To a suspension of (R)-1-(2,4-dimethoxybenzyl)-5-ethoxy-6-methyl-1,2,3,6-tetrahydropyrazine (23 g, 79 mmol, 1 eq) in MeTHF (46 mL), under N₂, was added formohydrazide (5 g, 83 mmol, 1.05 eq). The resulting mixture was heated at 65°C for 4-5 hours and then it was allowed to cool to room temperature, water was added and the organic layer was separated; the aqueous layer was extracted with MeTHF (2 × 100 mL). The combined organic layers were dried over Na₂SO₄, filtered and concentrated under reduced pressure to afford crude product as an orange viscous oil. Purification as described in Example 16 yielded 5.7 g (y = 36%) of the title compound as a colorless dense oil.

LC-MS and ¹H-NMR matched those of Example 16.

### Example 18

(R)-7-(2,4-dimethoxybenzyl)-8-methyl-5,6,7,8-tetrahydro-[1,2,4]triazolo[4,3-a]pyrazine

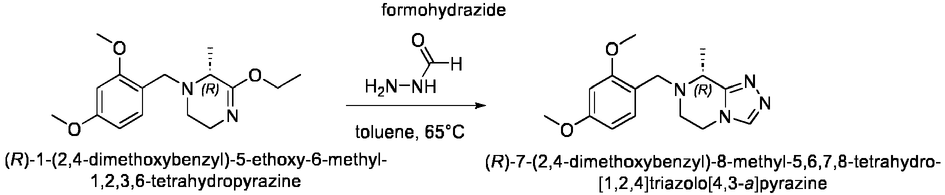

To a suspension of (R)-1-(2,4-dimethoxybenzyl)-5-ethoxy-6-methyl-1,2,3,6-tetrahydropyrazine (2.21 g; 7.57 mmol, 1 eq) in toluene (3.3 mL), under N₂ atmosphere, was added formohydrazide (476.8 mg; 87.95 mmol, 1.05 eq). The resulting mixture was heated at 65°C for 5 hours and then it was allowed to cool to the room temperature. It was diluted with toluene (20 mL) and poured into an aqueous solution of 1N HCl (20 mL). The two phases were separated, and the aqueous layer was washed with toluene

(1 × 20 mL). The aqueous phase was brought to pH 11 with 32% of NaOH and extracted with toluene (3 × 20 mL). The combined organic layers were dried over Na₂SO₄, filtered and concentrated under reduced pressure to afford crude product as an orange viscous oil. Purification as described in Example 16 yielded 830 mg (y = 38%) of the title compound as a colorless dense oil.

LC-MS and ¹H-NMR matched those of Example 16.

### Example 19

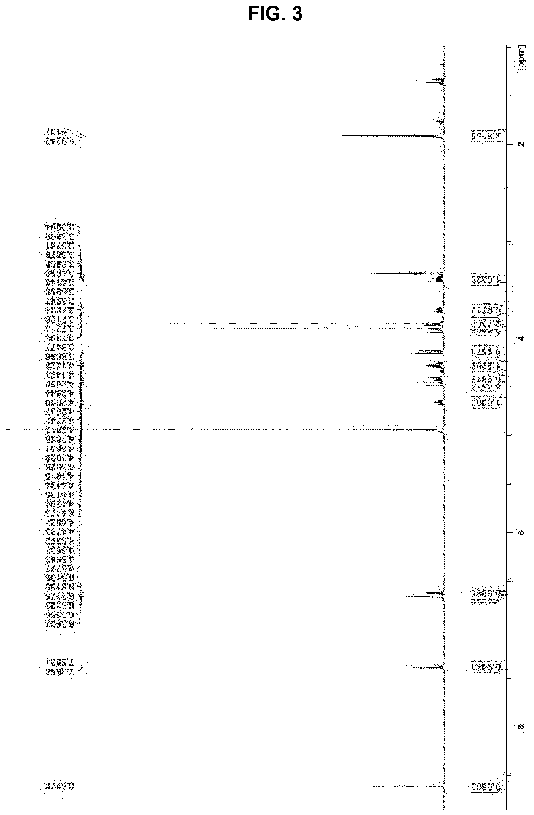

Compound between oxalic acid and (R)-7-(2,4-dimethoxybenzyl)-8-methyl-5,6,7,8-tetrahydro-[1,2,4]triazolo[4,3-a]pyrazine
(R)-7-(2,4-dimethoxybenzyl)-8-methyl-5,6,7,8-tetrahydro-[1,2,4]triazolo[4,3-a]pyrazine (1 g, 3.5 mmol, 1 eq) was dissolved in 4 mL of DCM at room temperature, then a solution of oxalic acid (1.56 g, 17.5 mmol, 5 eq) in EtOH (2 mL) was added and a precipitate was formed almost immediately. The reaction mixture was aged for 3 hours, then it was filtered washing with cold EtOH (1 mL). The solid was dried under vacuum at 40°C for 18 hours, yielding 1.24 g of the title compound as a crystalline white solid (assay of free base in the compound 53% by qNMR).
LC-MS: *m*/*z* = 289.4 (M+H)⁺
¹H NMR (500 MHz, MeOD): δ (ppm): 8.61 (s, 1H), 7.38 (d, *J* = 8.3 Hz, 1H), 6.66 (d, *J* = 2.3 Hz, 1H), 6.62 (dd, *J* = 8.4, 2.4 Hz, 1H), 4.66 (q, *J* = 6.7 Hz, 1H), 4.47 (d, *J* = 13.3 Hz, 1H), 4.41 (dt, *J* = 13.4, 4.5 Hz, 1H), 4.27 (m. 1H), 4.14 (d, *J* = 13.3 Hz, 1H), 3.90 (s, 3H), 3.85 (s, 3H), 3.71 (dt, *J* = 13.4, 4.5 Hz, 1H), 3.39 (ddd, *J* = 13.8, 9.4, 6.8 Hz, 1H), 1.92 (d, *J* = 6.8 Hz, 3H) - - reported in Fig. 3

The XRPD diffractogram acquired at room temperature with Cu-Kα radiation having a wavelength of 1.5419 Å is reported in Fig. 1.

### c) Synthesis of compounds of formula X where G=H

### Example 20

(R)-3-methyl-5-(8-methyl-5,6,7,8-tetrahydro-[1,2,4]triazolo[4,3-a]pyrazin-3-yl)-1,2,4-thiadiazole

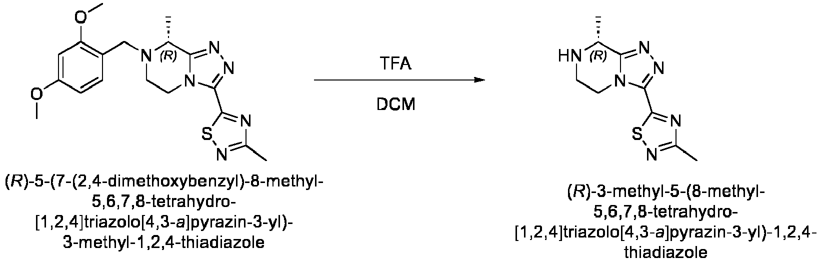

To a solution of (*R*)-5-(7-(2,4-dimethoxybenzyl)-8-methyl-5,6,7,8-tetrahydro-[1,2,4]triazolo[4,3-a]pyrazin-3-yl)-3-methyl-1,2,4-thiadiazole (20.23 g; 52.41 mmol, 1 eq.) in 120 mL of CH₂Cl₂, TFA (15.6 mL; 209.64 mmol, 4 eq.) was added dropwise at 0 °C, under N₂ atmosphere. The reaction mixture was allowed to warm to room temperature and was stirred for 24 h; during this time the color turned purple and a white precipitate was formed. The reaction was quenched by the addition of H₂O (150 mL) and the resulting mixture was stirred for further 30 min. The precipitate formed during the reaction was filtered off and the two phases were separated. The aqueous layer was washed with 150 mL of CH₂Cl₂, then it was brought to pH 11 with 32% of NaOH and it was extracted with CH₂Cl₂ (4 × 100 mL). The combined organic layers were dried over anhydrous Na₂SO₄, filtered and evaporated to dryness yielding 9.54 g (y = 77%) of the title compound as an off-white solid, which was used without further purification.
LC-MS: *m*/*z* = 237.2 (M+H)⁺

¹H NMR (500 MHz, MeOD): δ (ppm) 4.67 (dddd, *J* = 13.4, 4.6, 2.5, 0.8 Hz, 1H), 4.34 - 4.24 (m, 2H), 3.45 (ddd, *J* = 13.8, 5.0, 2.5 Hz, 1H), 3.21 (ddd, *J* = 13.7, 10.5, 4.6 Hz, 1H), 2.74 (s, 3H), 1.65 (d, *J* = 6.8 Hz, 3H).

### Example 21

(R)-3-methyl-5-(8-methyl-5,6,7,8-tetrahydro-[1,2,4]triazolo[4,3-a]pyrazin-3-yl)-1,2,4-thiadiazole

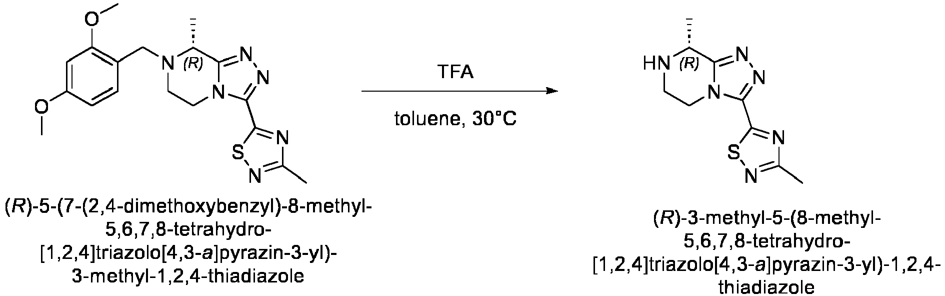

To a solution of (*R*)-5-(7-(2,4-dimethoxybenzyl)-8-methyl-5,6,7,8-tetrahydro-[1,2,4]triazolo[4,3-a]pyrazin-3-yl)-3-methyl-1,2,4-thiadiazole (3.52 g; 9.11 mmol; 1 eq.) in 21 mL of toluene, TFA (2.79 mL; 36.43 mmol; 4 eq.) was added dropwise at 0°C, under N₂ atmosphere, then the reaction mixture was heated at 30°C for 9 hours. 16 mL of H₂O were added and the mixture was stirred for further 60 min. The two phases were separated, the aqueous layer was washed with DCM (3 × 25 mL) to eliminate the precipitate formed during the reaction, then it was brought to pH 11 with 32% NaOH and it was extracted with DCM (3 × 25 mL). The combined organic layers were dried over Na₂SO₄, filtered and evaporated to dryness, yielding 902 mg (y = 42%) of the title compound as a light-brown solid.

LC-MS matched that of Example 20.

### Example 22

(R)-3-methyl-5-(8-methyl-5,6,7,8-tetrahydro-[1,2,4]triazolo[4,3-a]pyrazin-3-yl)-1,2,4-thiadiazole

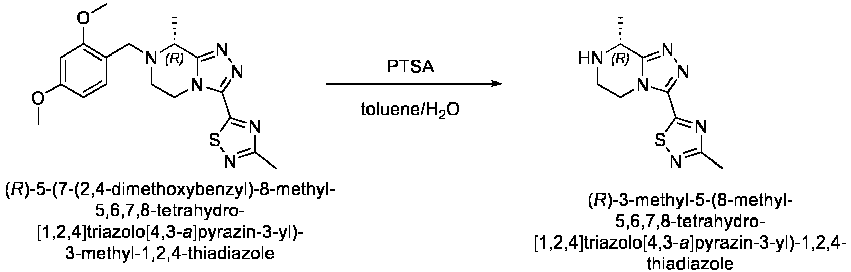

To a solution of (*R*)-5-(7-(2,4-dimethoxybenzyl)-8-methyl-5,6,7,8-tetrahydro-[1,2,4]triazolo[4,3-*a*]pyrazin-3-yl)-3-methyl-1,2,4-thiadiazole (2.44 g; 6.31 mmol; 1 eq.) in 24.5 mL of toluene, were added water (12.2 mL) and 4-methylbenzenesulfonic acid monohydrate (2.40 g, 12.62 mmol, 2 eq.) at room temperature, under N₂ atmosphere. The resulting biphasic mixture was heated at 85 °C for 2 h, under vigorous stirring, then it was allowed to cool to room temperature and 12 mL of water were added. The precipitate formed during the reaction was filtered off and the two phases were separated. The acidic aqueous layer was washed with 20 mL of toluene, then NaOH 32% was added until pH > 11 and the aqueous layer was extracted with DCM (3 × 35 mL). The combined organic layers were evaporated to dryness under reduced pressure, yielding 850mg (y = 57%) of the title compound as an off-white solid.

LC-MS matched that of Example 20.

### d) Synthesis of compounds of formula X where G=-CO-Ar¹ from compounds of formula X where G=H

### Example 23

(R)-(4-fluorophenyl)(8-methyl-3-(3-methyl-1,2,4-thiadiazol-5-yl)-5,6-dihydro-[1,2,4] triazolo[4,3-a]pyrazin-7(8H)-yl)methanone

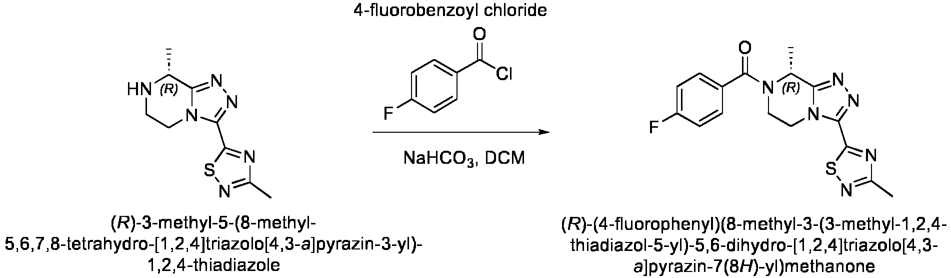

To a solution of (R)-3-methyl-5-(8-methyl-5,6,7,8-tetrahydro-[1,2,4]triazolo[4,3-a]pyrazin-3-yl)-1,2,4-thiadiazole (19.6 g, 83 mmol, 1 eq) in DCM/NaHCOs sat (400 mL, 3/1 respectively) at room temperature was added 4-fluorobenzoyl chloride (13.8 g, 87 mmol, 1.05 eq) in about 10 minutes. The resulting mixture was then stirred for 1 hour at room temperature and the layers were separated. The organic phase was washed with 1N HCl (100 mL), then was dried over Na₂SO₄, filtered and concentrated to dryness under reduced pressure to afford an off-white solid, which was crystallized from a H₂O/EtOH mixture, yielding 24 g (y = 81%) of the title compound as a white solid.
LC-MS: *m*/*z* = 359.3 (M+H)⁺
¹H NMR (500 MHz, DMSO): δ (ppm) 7.60 (m, 2H), 7.33 (m, 2H), 6.19-5.32 (bm, 1H), 4.68 (dd, *J* = 13.4, 3.5 Hz, 1H), 4.30 (m, 1H), 4.19-3.81 (bm, 1H), 3.64 (s, 1H), 2.69 (s, 3H), 1.62 (d, *J* = 6.9 Hz, 3H).

¹³C NMR (126 MHz, DMSO): δ (ppm) 175.00, 174.11, 169.27, 162.32, 154.70, 145.44, 132.17, 129.97, 116.19, 45.28, 40.50, 19.14.

¹⁹F NMR (471 MHz, DMSO): δ (ppm) -110.48.

### e) Alternative synthesis of compounds of formula XIII

### Example 24

(R)-8-methyl-5,6,7,8-tetrahydro-[1,2,4]triazolo[4,3-a]pyrazine 2,2,2-trifluoroacetate (common starting intermediate for the other synthesis in this paragraph)

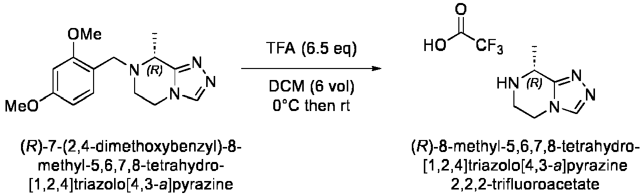

To a solution of (R)-7-(2,4-dimethoxybenzyl)-8-methyl-5,6,7,8-tetrahydro-[1,2,4]triazolo[4,3-a]pyrazine (860 mg; 2.98 mmol; 1 eq.) in 5.6 mL of DCM, TFA (1.46 mL; 19.03 mmol; 6.5 eq.) was added dropwise at 0°C, under N₂ atmosphere. Then the reaction mixture was allowed to warm to room temperature and was stirred for 3 h. 5 mL of H₂O were added and the mixture was stirred for further 30 min. The precipitate formed during the reaction was filtered off and the two phases were separated. The aqueous phase was evaporated to dryness under vacuum, affording 665 mg (y = 88%) of the title compound as a yellowish oil, used without further purification.
LC-MS: *m*/*z* = 139.1 (M+H)⁺
¹H NMR (500 MHz, DMSO): δ (ppm) 8.58 (s, 1H), 4.73 (m, 1H), 4.35 (m, 1H), 4.14 (m, 1H), 3.71 (m, 1H), 3.48 (m, 1H), 1.64 (d, *J* = 6.7 Hz, 3H).

### Example 25

(R)-(4-fluorophenyl)(8-methyl-5,6-dihydro-[1,2,4]triazolo[4,3-a]pyrazin-7(8H)-yl) methanone

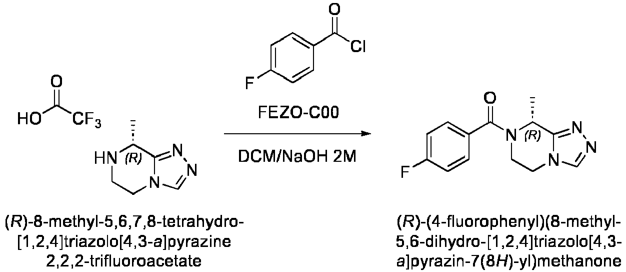

(R)-8-methyl-5,6,7,8-tetrahydro-[1,2,4]triazolo[4,3-a]pyrazine 2,2,2-trifluoroacetate (752 mg, 2.98 mmol, 1.0 eq) was dissolved in a mixture of DCM (10 mL) and NaOH 2M (aq. solution, 5 mL). The biphasic mixture was vigorously stirred for 10 minutes, then 4-fluorobenzoyl chloride (577 mg, 3.64 mmol, 1.2 eq) was added dropwise. The reaction mixture was stirred for 1 h at room temperature, then the two layers were separated. The aqueous layer was extracted with DCM (3 × 10 mL), then the combined organic phases were evaporated to dryness, affording 168 mg (y = 22%) of the title compound as an off-white powder used without further purification.
LC-MS: *m*/*z* = 261.2 (M+H)⁺
¹H NMR (500 MHz, DMSO): δ (ppm) 8.50 (s, 1H), 7.57 (m, 2H), 7.32 (m, 2H), 4.17 (m, 2H), 4.04 (td, *J* = 12.2, 4.2 Hz, 2H), 3.57 (s, 1H), 1.54 (d, *J* = 6.9 Hz, 3H).

13C NMR (126 MHz, DMSO-d6) □ (ppm): 169.29, 162.24, 151.18, 142.82, 132.38, 129.91, 116.19, 62.97, 42.92.

### Example 26

Benzyl (R)-8-methyl-5,6-dihydro-[1,2,4]triazolo[4,3-a]pyrazine-7(8H)-carboxylate

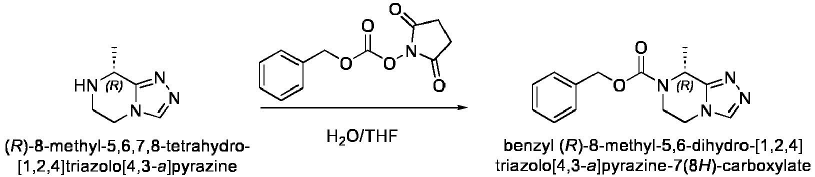

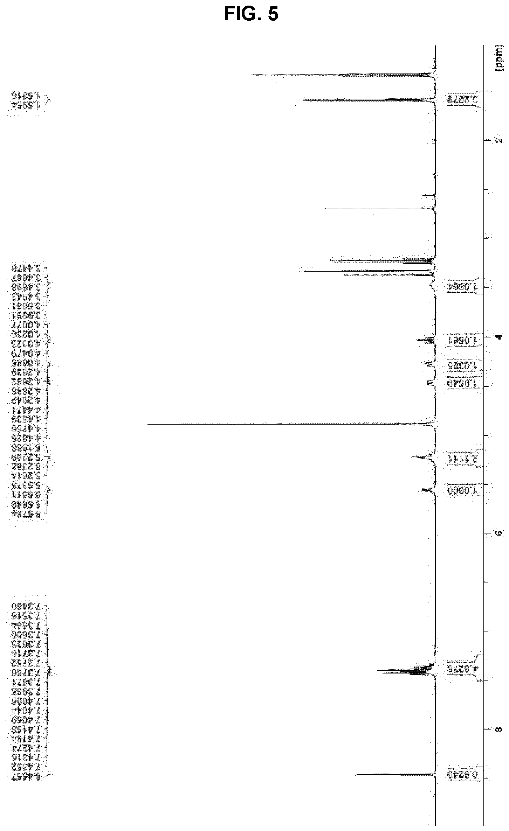

(R)-8-methyl-5,6,7,8-tetrahydro-[1,2,4]triazolo[4,3-a]pyrazine (2.25 g, 16.30 mmol, 1.0 eq) was dissolved in a mixture of H₂O/THF 1:1 (14 mL), then benzyl (2,5-dioxopyrrolidin-1-yl) carbonate was added (4.48 g, 17.99 mmol, 1.1 eq). The reaction mixture was stirred for 3 hours and 30 minutes at room temperature, then DCM was added (10 mL) and the two layers were separated. The organic phase was evaporated to dryness and the resulting crude product was purified by flash column chromatography eluting with EtOAc:Methanol (80:20), yielding 4.17 g (y = 94%) of the title compound as a pale yellow oil.
LC-MS: *m*/*z* = 273.3 (M+H)⁺
¹H NMR (500 MHz, DMSO): δ (ppm) 8.45 (s, 1H), 7.43-7.35 (m, 5H), 5.56 (q, *J* = 6.8 Hz, 1H), 5.26-5.20 (m, 2H), 4.46 (dd, *J* = 14.3, 3.5 Hz, 1H), 4.28 (dd, *J* = 12.5, 2.7 Hz, 1H), 4.03 (td, *J* = 12.1, 4.3 Hz, 1H), 3.50-3.45 (m, 1H), 1.59 (d, *J* = 6.9 Hz, 3H) - reported in Fig. 5.

## Claims

1. A process for the preparation of compound of formula X: where 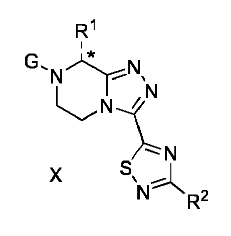
G is
- a -X-Ar¹ group, where X is a carbonyl group CO and Ar¹ is an optionally substituted 5- or 6-membered aryl or heteroaryl group, or
- a nitrogen protecting group (PG), or
- a hydrogen;
R¹ is an optionally substituted C₁-C₆ alkyl;
R² is an optionally substituted C₁-C₆ alkyl; or optionally substituted phenyl group; and
*--- represents one enantiomer R or S of compounds of formula X or a mixture of the two enantiomers in any proportion including the racemate;
the process including the following step: 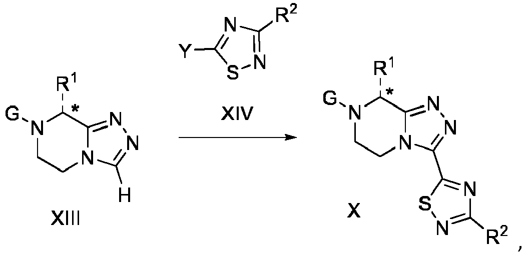 reacting a compound of formula XIII with a compound of formula XIV in a solvent in the presence of one or more metal-based catalysts, optionally in the presence of one or more ligands and/or one or more bases, to obtain a compound X, where Y is a leaving group.

2. Process according to claim 1, wherein R¹ and R² are methyl.

3. Process according to claims 1 or 2, wherein G is a nitrogen protecting group (PG) selected from an optionally substituted carbamate group, optionally substituted alkyl group, optionally substituted benzyl group, optionally substituted amido group.

4. Process according to any one of claims 1 to 3, wherein G is selected from dimethoxybenzyl (DMB), t-butoxycarbonyl (Boc), benzyloxycarbonyl (Cbz) or p-fluorobenzoyl.

5. Process according to any one of claims 1 to 4, wherein Y is selected from an halogen such as Cl, Br or I, a sulfonate group such as CH₃SO₂-O-, pToISO₂-O-, CF₃SO₂-O- or other C₁-C₈ alkyl- or C₆-C₁₀ aryl-sulfonates, a sulfone group such as CH₃SO₂-, pToISO₂-, CF₃SO₂- or other C₁-C₈ alkyl- or C₆-C₁₀ aryl -sulfones, a sulfoxide group such as CHsSO-, pToISO-, CF₃SO- or other C₁-C₈ alkyl- or C₆-C₁₀ aryl -sulfoxides, a sulfinate group such as CH₃SO-O-, pToISO-O-, CFsSO-O- or other C₁-C₈ alkyl- or C₆-C₁₀ aryl-sulfinates, and the rest of the symbols, G, R¹, R² and " *--- ", have the same meaning as described above.

6. Process according to any one of claims 1 to 5, wherein metal-based catalysts is selected from palladium, copper, rhodium, nickel, ruthenium, iridium, iron or manganese based catalyst or mixtures thereof.

7. Process according to any one of claims 1 to 6, wherein the metal-based catalysts is selected from palladium, copper based catalyst, or a mixture thereof.

8. Process according to any one of claims 1 to 7, wherein the ligands, if present, are selected from substituted phosphines or other compounds of phosphorous, such as triphenylphosphine, tri-tolylphosphine, tributylphosphine, tricyclohexylphosphine, 2,2'-Bis(diphenylphosphino)-1,1 '-binaphthyl (*BINAP*), 4,5-Bis(diphenylphosphino)-9,9-dimethylxanthene (Xantphos), 1,1'-bis(diphenylphosphino)ferrocene (*dppf*), bis(diphenylphosphino)ethane (*dppe*), bis[(2-diphenylphosphino)phenyl] ether (*DPEphos*), 1,2-bis-2,5-dimethylphospholane (*Me-DuPhos*), 1,2-bis(2,5-diphenylphospholano)ethane (*Ph-BPE*) dicyclohexyl(2',6'-diisopropoxy-[1,1'-biphenyl]-2-yl)phosphine (*RUphos*); or ligands based on carbon-carbon double bonds or aromatic rings, such as ethylene, propene, cyclooctadiene, cyclooctatetraene, cyclopentadiene, pentamethylcyclopentadiene, benzene, toluene, mesitylene, *p*-cymene, hexamethylbenzene, 2,5-norbornadiene, 1,5-hexadiene; or ligands based on pyridine moieties such as 2,2'-bispyridine, 2-(2-pyridinyl)quinoline, 1,10-phenantroline, ethylenediamine moieties such as ethylenediamine, N,N'-dimethylethylenediamine, 1,2-diphenylethlenediamine, N,N'-dicyclohexylethylenediamine, nitrile moieties such as benzonitrile, acetonitrile, dione moieties such as optionally substituted acetylacetones, ether moieties such as ethylene glycol dimethylether, diethylene glycol dimethylether, or N-heterocyclic carbenes such as deriving from imidazolium, triazolium, pyrazolium, oxazolium salts, or optionally substituted porphyrin systems.

9. Process according to any one of claims 1 to 8, wherein the base, if present, is selected from inorganic bases, such as potassium, sodium or cesium carbonate, sodium or potassium bicarbonate, sodium, potassium or cesium hydroxide, sodium or potassium phosphates or hydrogenophosphates or organic bases such as trialkylamines, n-methylimidazole, optionally substituted pyridines, 1,5-diazabiciclo(5.4.0)undec-7-ene (*DBU*)*,* 1,5-Diazabicyclo 4.3.0 non-5-ene (*DBN*), 1,4-diazabicyclo[2.2.2]octane (*DABCO*) and phosphazenes or mixtures thereof.

10. Process according to any one of claims 1-9, wherein the process further includes one or more of the following steps, the symbols PG, R¹, R² and " *---" having the same meaning as defined in claim 1:
- a step to prepare a compound of formula XIII by reacting a compound of formula XII with formohydrazide: 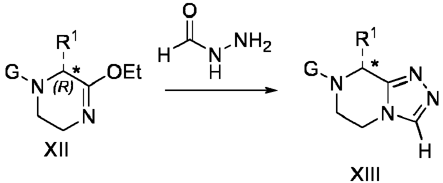
- a step to prepare compound of formula X where G is hydrogen starting from a compound of formula X where G = PG: 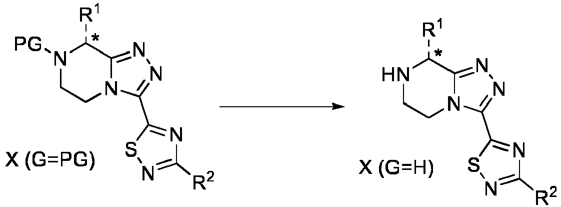
- a step to to prepare compound of formula X where G is -CO-Ar¹ starting from a compound of formula X where G = H:

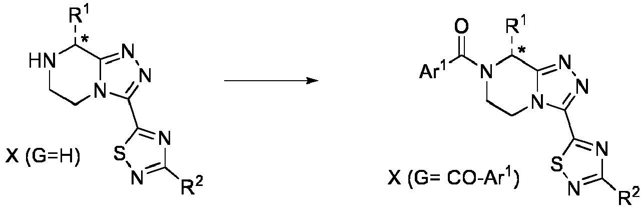

11. Process according to any one of claims 1-10, wherein compound of formula X is fezolinetant, 4-fluorophenyl)-[(8R)-8-methyl-3-(3-methyl-1,2,4-thiadiazol-5-yl)-6,8-dihydro-5H-[1,2,4]triazolo[4,3-a]pyrazin-7-yl]methanone.

12. A compound of formula XV or a salt and/or cocrystal thereof: 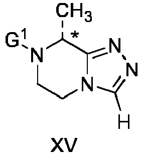 where
G¹ is
- a 4-fluorobenzoyl group; or
- a nitrogen protecting group chosen between optionally substituted carbamate groups including tert-butoxycarbonyl and benzyloxycarbonyl groups, benzyl and 2,4-dimethoxybenzyl;
*--- represents one enantiomer R or S of compounds of formula XV or a mixture of the two enantiomers in any proportion including the racemate.

13. A complex between oxalic acid and a compound of formula XV as defined in claim 12, in which G¹ is 2,4-dimethoxybenzyl and "*--" represent the R enantiomer as major isomer.

14. Complex according to claim 13 in crystalline form.

15. Complex according to claim 14 in a crystal form **characterized by** having a powder X-ray diffractogram comprising peaks at 5.8, 9.0, 12.2, 13.5, 25.6 and 26.5 ± 0.2 degrees 2-Theta, when measured at room temperature with Cu-Kα radiation having a wavelength of 1.5419 Å.
